# EUROPÄISCHE PATENTSCHRIFT

(11) **EP 1 417 926 B1**
(45) Veröffentlichungstag und Bekanntmachung des Hinweises auf die Patenterteilung: **19.12.2007**
(21) Anmeldenummer: 02405969.3
(22) Anmeldetag: 11.11.2002
(51) Int. Cl.: A61B 5/0265

(54) **Verfahren und Vorrichtung zum Erfassen und Bearbeiten eines EKG-Signals**
Method and apparatus for ECG signal detection and processing
Méthode et appareil pour l'enrégistrement et le traitement des signaux ECG

(43) Veröffentlichungstag der Anmeldung: 12.05.2004
(73) Patentinhaber: Schiller AG, 6340 Baar (CH)
(72) Erfinder: Schmid, Johann-Jakob, 8911 Rifferswil (CH); Abächerli, Roger, 6312 Steinhausen (CH); Felblinger, Jacques, Prof., 54710 Ludres (FR)
(74) Vertreter: Müller, Christoph Emanuel

(56) Entgegenhaltungen:
- AT-B- 399 646
- DE-A- 4 429 335
- US-A- 3 809 070
- US-A- 4 412 545
- US-A- 5 935 077
- US-A- 6 132 380

## Beschreibung

Die Erfindung betrifft ein Verfahren und eine Vorrichtung zum Erfassen und Bearbeiten eines EKG-Signales, wobei eine nichtinvasiven Messung des Blutflusses erfolgen kann, mit den Merkmalen des Oberbegriffs der unabhängigen Patentansprüche. Ein derartiges Verfahren und eine solche Vorrichtung sind aus AT-B-399 646 bekannt.

Die Grösse des Blutflusses in Gefässen und insbesondere der Blutausstoss des Herzen (cardiac output) ist im Zusammenhang mit vielen Diagnosen relevant. Ebenso ist die Aufnahme von Elektrokardiogrammen (EKG) für diagnostische Zwecke weit verbreitet. Bestimmung von Blutfluss und/oder die Aufnahme von Elektrokardiogrammen kann insbesondere auch während der Aufnahme von Kernspintomographien hilfreich sein. Elektrokardiogramme werden beispielsweise zur Überwachung von Patienten während Kernspintomographien verwendet. Aufgrund des in Kernspintomographen herrschenden starken magnetischen Feldes und der eingesetzten Hochfrequenzsignale werden EKG-Signale aber häufig gestört. Insbesondere überlagern sich dem EKG-Signal Störsignale. Verfahren zum Verhindern von solchen Störsignalen und zum Übermitteln des EKG-Signals aus dem abgeschirmten Inneren einer Kernspintomographie (im folgenden MRI) Anordnung sowie dafür ausgebildete Elektroden sind beispielsweise aus EP 1 050 270, US 6 073 030, US 6 032 063, US 6 052 614, EP 487 441, EP 695 139, US 5 733 247, WO 92/21286, FR 2 685 968, US 4 991 580 oder EP 132 785 bekannt. Diese verschiedenen Verfahren befassen sich insbesondere mit aufgrund des starken Magnetfeldes und der Hochfrequenzsignale induzierten Störungen am elektrischen Signal.

Es ist eine Aufgabe der vorliegenden Erfindung, ein Verfahren und ein Vorrichtung zu schaffen, mit dem sich EKG-Signale auch bei hohen Magnetfeldern präzise erfassen lassen. Eine weitere Aufgabe der vorliegenden Erfindung besteht darin, ein Verfahren und eine Vorrichtung zum nichtinvasiven Messen des cardiac output zu schaffen.

Erfindungsgemäss wird die Aufgabe mit einem Verfahren und mit einer Vorrichtung mit den Merkmalen des kennzeichnenden Teils der unabhängigen Patentansprüche gelöst. Die Erfinder der vorliegenden Anmeldung haben herausgefunden, dass aufgrund des Halleffektes im Bereich von Blutgefässen aufgrund der Bewegung des Blutes Potentialdifferenzen entstehen, wenn das Blut einem verhältnismässig starken Magnetfeld ausgesetzt sind. Eine bevorzugte Ausführungsform der Erfindung beruht auf der Anwendung dieser Erkenntnis zum nichtinvasiven Messen des Blutflusses in einem Blutgefäss eines Körpers eines Lebewesens. Der Körper wird dazu einem ausreichend starken Magnetfeld ausgesetzt. An der Körperoberfläche werden an wenigstens zwei Stellen Ableitungen eines elektrischen Potentials vorgenommen. Die Stellen sind vorzugsweise benachbart zu dem Blutgefäss, in welchem der Blutfluss ermittelt werden soll. Der Blutfluss wird auf der Grundlage der Potentialdifferenz zwischen den wenigstens zwei Stellen ermittelt. Die Ermittlung beruht auf der Erkenntnis, dass die Potentialdifferenz aufgrund des Halleffektes proportional zur Bewegung von Ladungsträgern im Blut im Gefäss und daher proportional zum Blutfluss ist. Damit lässt sich der Blutfluss zumindest quantitativ ermitteln.

Ladungsträger sind beispielsweise Hämoglobin oder im Blut enthaltene Ionen. Die Messung ist optimal, wenn das Gefäss senkrecht zum Hauptmagnetfeld verläuft.

Bevorzugt wird im vorliegenden Verfahren das Magnetfeld durch eine MRI-Anordnung erzeugt. MRI-Anordnungen erzeugen typischerweise Magnetfelder im Bereich von 0,5 bis 3 Tesla. In diesem Fall ermöglicht das bevorzugte Verfahren die Bestimmung des Blutflusses gleichzeitig zu der Vornahme einer MRI-Diagnose.

Dank der MRI Diagnose kann gleichzeitig der Querschnitt des Blutgefässes bestimmt werden. Bei Kenntnis des Querschnitts lässt sich der Blutfluss auch quantitativ bestimmen.

Besonders bevorzugt erfolgt die Messung der Potentialdifferenz mit einem an sich herkömmlichen EKG-Gerät, welches zur Aufnahme, Bearbeitung und Übermittlung von EKG-Signalen auch unter Einfluss von starken Magnetfeldern ausgebildet ist.

Es sind auch andere Geräte zum Messen von elektrophytologischen Parametern denkbar, z.B. EEG etc. Hier und im Folgenden wird zur Illustration auf EKG-Geräte Bezug genommen. Andere Geräte sind ebenso verwendbar.

Gemäss einem weiteren bevorzugten Aspekt der vorliegenden Erfindung werden die wenigstens zwei Elektroden des EKG-Gerätes am Körper des Lebewesens derart angelegt, dass aus der gemessenen Potentialdifferenz der cardiac output des Herzens bestimmt werden kann.

Gemäss der Erfindung wird die Erkenntnis zur Erfassung und Bearbeitung eines EKG-Signales angewandt. Die Erfinder haben festgestellt, dass bei der Aufnahme von Elektrokardiogrammen während einer Kernspintomographie dem EKG-Signal ein Signalteil überlagert wird, der auf eine Potentialdifferenz zurückgeht, die aufgrund des Halleffekts durch im Blut enthaltene Ladungsträger erzeugt wird. Gemäss der Erfindung wird deshalb ein Abschnitt des Signalverlaufes ermittelt, welcher aufgrund des Halleffekts durch Ladungsträger im durch das Herz ausgestossene Blut erzeugt wird. Anschliessend wird das EKG-Signal bearbeitet. Der durch den Halleffekt bewirkte Teil des EKG-Signales wird eliminiert. Dadurch lässt sich auch in einem starken Magnetfeld, wie es beispielsweise in einer MRI-Anordnung herrscht, ein brauchbares EKG-Signal ermitteln.

Gemäss einem bevorzugten Ausführungsbeispiel erfolgt auf der Grundlage des Signalverlaufs im ermittelten Abschnitt des EKG-Signals, insbesondere aufgrund der Amplitude in diesem Abschnitt, eine Bestimmung des cardiac output des Lebewesens.

Die erfindungsgemässe Vorrichtung dient zum Erfassen und Bearbeiten eines EKG-Signals. Die Vorrichtung ist insbesondere zur Durchführung eines der vorstehend beschriebenen Verfahren geeignet. Die Vorrichtung weist eine EKG-Anordnung mit wenigstens zwei Elektroden zum Messen einer Potentialdifferenz zwischen wenigstens zwei Stellen an der Oberfläche des Körpers eines Lebewesens auf. Die EKG-Anordnung ist im Wesentlichen herkömmlich ausgebildet, und ist zur Aufnahme von EKG-Signalen auch unter Einfluss von starken Magnetfeldern und Hochfrequenzsignalen geeignet.

Der erfindungsgemässen Vorrichtung können ausserdem Mittel zum Erzeugen eines Magnetfeldes zugeordnet sein. Typischerweise sind diese Mittel durch eine Spule einer Kernspintomographie-Anordnung gebildet. Die Vorrichtung ist ausserdem mit Mitteln zum Ermitteln eines durch die Bewegung von Ladungsträgern im Gefäss aufgrund des Halleffekts erzeugten Anteils des durch die EKG-Anordnung erzeugten Potentialsignals versehen. Diese Mittel können besonders bevorzugt softwaremässig in einem herkömmlichen EKG-Gerät implementiert sein. Die erfindungsgemässe Vorrichtung ist dann ein herkömmliches EKG-Gerät mit Zusatzfunktionen.

Die Erfindung wird im Folgenden in Ausführungsbeispielen und an Hand der Zeichnungen näher erläutert. Es zeigen:
- Figur 1: eine schematische Darstellung einer erfindungsgemässen Vorrichtung,
- Figur 2: eine schematische Darstellung des Verlaufs eines EKG-Signals,
- Figur 3: eine schematische Darstellung der Messung des Blutflusses in einem Gefäss,
- Figur 4: eine schematische Darstellung der Anordnung von Elektroden zur Bestimmung des cardiac output und
- Figuren 5a und 5b: Darstellung eines innerhalb und ausserhalb eines Magnetfeldes aufgenommenen EKG-Signals

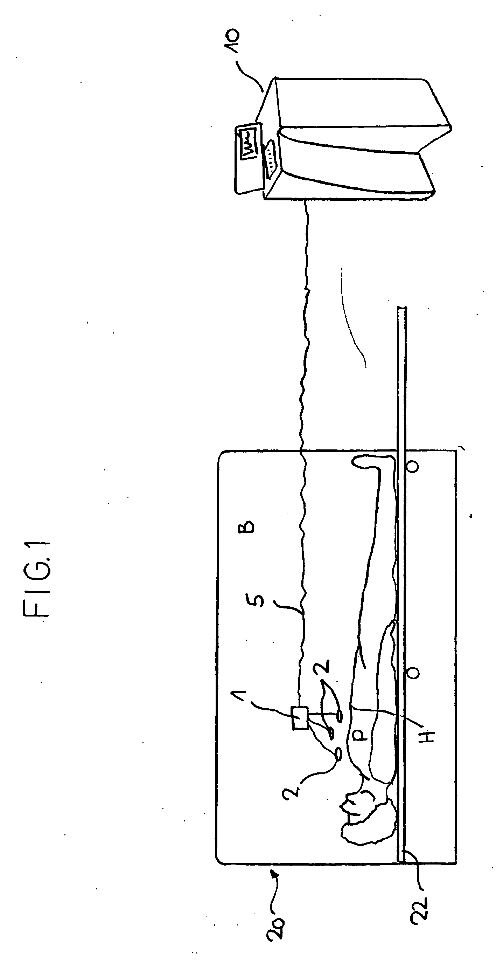

In Figur 1 ist schematisch eine erfindungsgemässe Vorrichtung zum nichtinvasiven Messen des Blutflusses und zur Aufnahme und Bearbeitung eines Elektrokardiogramms gezeigt. Die Vorrichtung besteht im Wesentlichen aus einer EKG-Anordnung 1, welche über ein Kabel 5 mit einer Auswert- und Anzeigeeinheit 10 verbunden ist. Es sind auch Übertragungen via Funk oder auf optischem Weg denkbar. Die EKG-Anordnung 1 ist im Inneren einer MRI Anordnung 20 angeordnet. Im Inneren der MRI-Anordnung 20 herrscht ein Magnetfeld B von grosser Stärke, typischerweise ein 0,5 bis 3 Tesla. Ein Patient P kann auf einem verschiebbar gelagerten Bett 22 in den nicht näher dargestellten Tunnel der MRI-Anordnung 20 bewegt werden. Die MRI-Anordnung 20 dient zur Aufnahme einer Kernspintomographie und ist in an sich bekannter Weise ausgebildet. Zum Messen des Blutflusses und zum Aufnehmen von EKG's ist die EKG-Anordnung mit Elektroden 2 versehen, welche auf der Haut H des Patienten P positioniert werden.

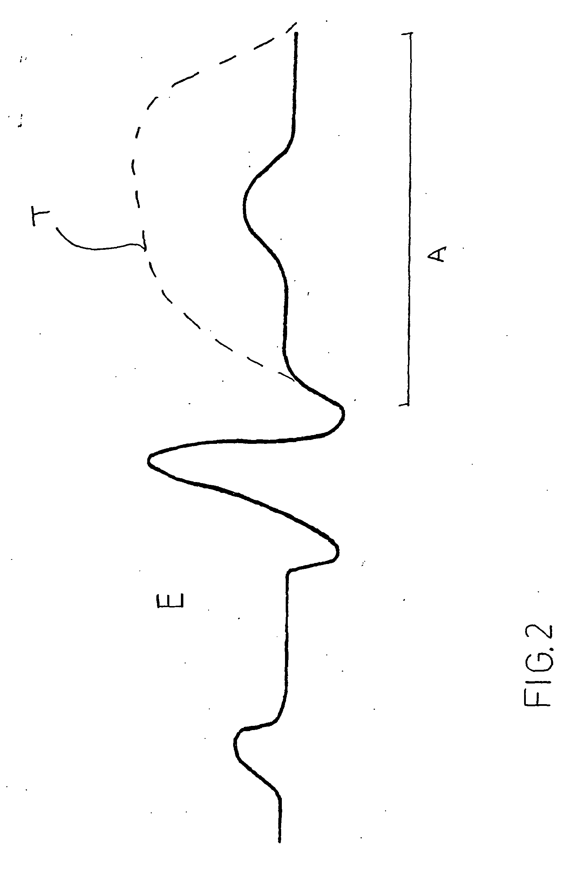

In Figur 2 ist der Verlauf eines EKG-Signals gezeigt, welches mit der erfindungsgemässen Vorrichtung aufgenommen wird. In durchgezogener Linie ist ein übliches EKG-Signal E gezeigt. Das EKG-Signal E ist beispielsweise mit der EKG-Anordnung erzielt worden, wenn die MRI-Anordnung 20 nicht in Betrieb ist, d.h. wenn kein starkes Magnetfeld B herrscht. In gestrichelter Linie ist in Figur 2 ein Abschnitt A des EKG-Signals gezeigt, welcher aufgrund des Halleffektes durch das Magnetfeld B bedingt ist. Sobald beim Betrieb der MRI-Anordnung ein EKG aufgenommen werden soll, wird dem eigentlichen EKG-Signal E ein auf den Halleffekt zurückgehender Signalteil T überlagert. Der Signalteil T ist für das Elektrokardiogramm störend. Es wird deshalb angestrebt, diesen Signalteil zu eliminieren. Die Vorrichtung enthält deshalb Mittel zum Ermitteln des Abschnitts A und zum Eliminieren des auf den Halleffekt zurückgehenden Anteils T der EKG-Kurve E. Diese Mittel sind typischerweise in der Anzeige und Auswerteinheit 10 angeordnet. Es ist aber auch denkbar, die Mittel softwaremässig in einer herkömmlichen EKG-Anordnung zu implementieren.

Der parasitäre Teil der EKG-Kurve kann beispielsweise durch Template Matching auf dem Fachmann an sich bekannte Art und Weise ermittelt werden.

Der durch den Halleffekt bewirkte Teil T der EKG-Kurve E kann aber auch für weitere Zwecke ausgewertet werden. Im Blut sind Ladungsträger enthalten. Aufgrund des starken Magnetfeldes während einer Kernspintomographie wird wegen des Halleffektes bei strömendem Blut eine Potentialdifferenz erzeugt. Die Amplitude des auf den Halleffekt zurückgehenden Abschnittes T der EKG-Kurve E ist daher ein Mass für den Blutfluss im entsprechenden Zeitpunkt. Die Amplitude im Abschnitt A ist daher ein Mass für den Fluss des Blutes aus dem Herzen während der Kontraktion, also ein Mass für den cardiac output. Die Menge des ausgestossenen Blutes entspricht dem Integral des auf den Halteffekt zurückgehenden Signalteils.

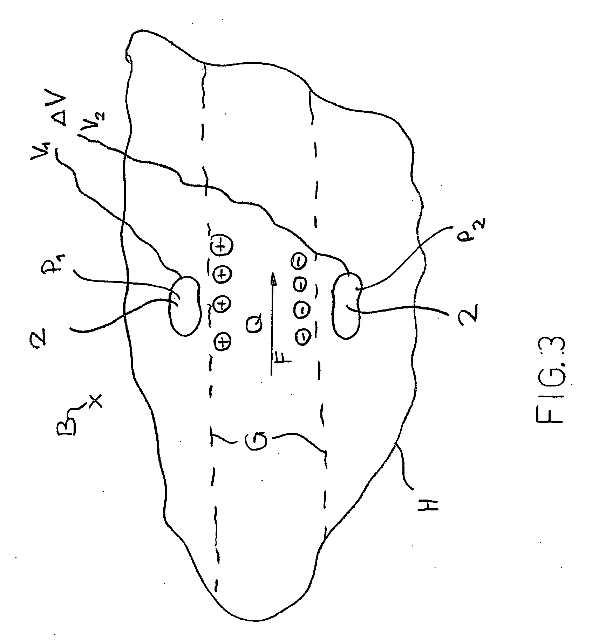

Figur 3 zeigt schematisch die Messung des Blutflusses F in einem beliebigen Blutgefäss G des Patienten P. Im Blut sind Ladungsträger Q enthalten. Aufgrund des Blutflusses in der Richtung F und des Magnetfeldes B (in Figur 3 in die Seite zeigend) ergibt sich eine Potentialdifferenz ΔV zwischen zwei Stellen P1, P2 im Gefäss G. Positive Ladungsträger bewegen sich auf die in Figur 3 obere Seite des Gefässes G, negative Ladungsträger auf die untere Seite des Gefässes G. Durch zwei benachbart zum Gefäss platzierte Elektroden 2 lässt sich das Potential V1, V2 an den Stellen P1, P2 und damit die Potentialdifferenz ΔV bestimmen. Die Potentialdifferenz ΔV ist proportional zum Blutfluss.

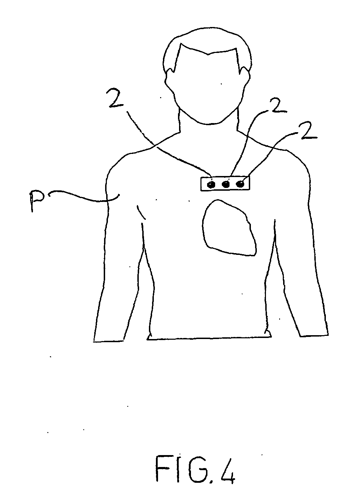

In Figur 4 ist schematisch dargestellt, an welchen Stellen des Körpers Elektroden zur Aufnahme eines EKG's und zur Bestimmung des cardiac output angelegt werden. Um den cardiac output zu ermitteln, werden wenigstens zwei, typischerweise drei Elektroden 2 in der in Fig. 4 gezeigten Anordnung auf dem Körper des Patienten P plaziert. Dadurch wird der Blutfluss in der Aorta, d. h. der cardiac output ermittelt. Andere Geräte wie z.B. EEG, EOG oder EMG können in gleicher Weise eingesetzt werden.

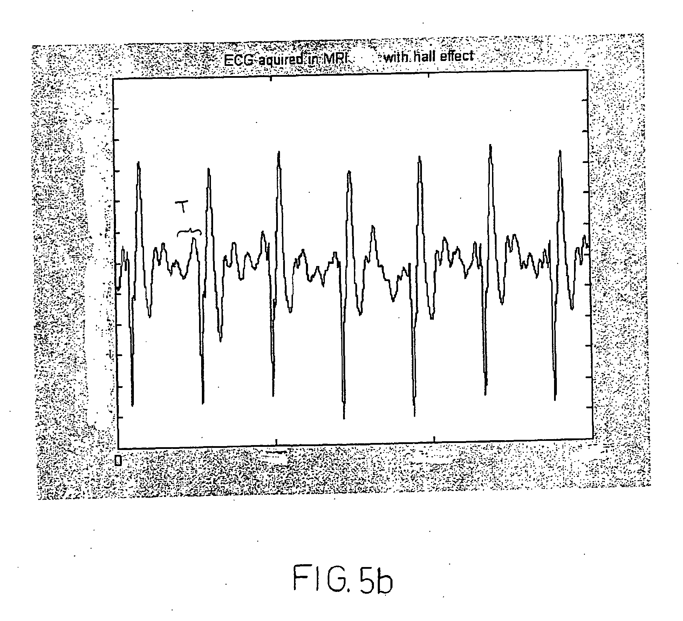

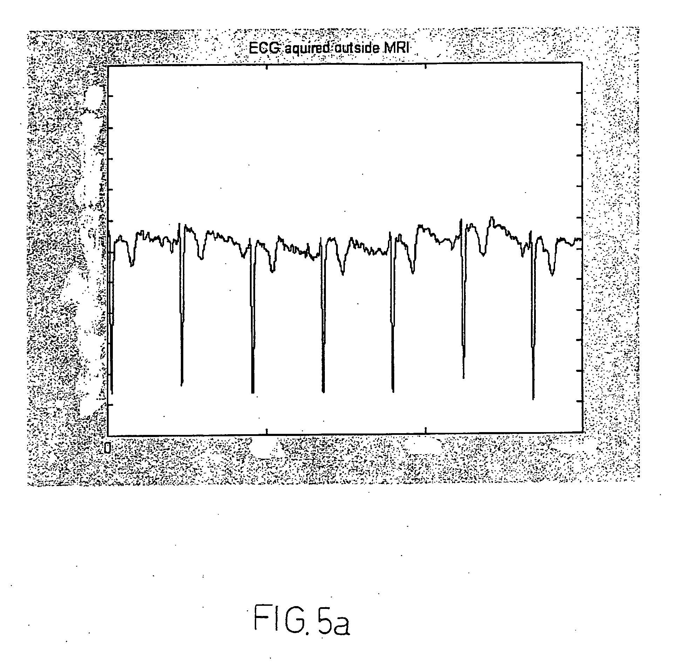

Figuren 5a und 5b zeigen den Effekt, den ein starkes Magnetfeld auf den Verlauf eines EKG-Signals aufgrund des Halleffekts hat anhand eines Beispiels.

Figur 5a zeigt den üblichen Verlauf eines EKG-Signals. Im Gegensatz zu Figur 5a wurde das Signal gemäss Figur 5b unter Einfluss eines Magnetfeldes innerhalb einer MRI-Vorrichtung aufgenommen. Dem EKG-Signal sind durch den Halleffekt erzeugte Signalanteile T überlagert. Diese Signalanteile geben zumindest ein qualitatives, bei Kenntnis des Querschnitts des entsprechenden Blutgefässes auch ein quantitatives Mass für den Blutfluss im Blutgefäss, also im Fall eines EKG's beispielsweise in der Aorta.

## Patentansprüche

1. Verfahren zum Erfassen und Bearbeiten eines EKG-Signales (E) eines Patienten (P), der einem Magnetfeld (B) ausgesetzt ist, bestehend aus den Schritten
- Aufnehmen eines Signals (E)
- Ermitteln eines Abschnittes (A) des Signalverlaufs, in welchem dem Signal (E) ein Signalanteil (T) überlagert ist, welcher aufgrund des Halleffektes durch Ladungsträger (Q)im durch das Herz ausgestossenen Blut erzeugt ist, und
- Bearbeiten des Signals (E), **gekennzeichnet durch** Eliminieren des **durch** den Halleffekt bewirkten Signalanteils (T) des Signals (E) im ermittelten Abschnitt (A).

2. Verfahren nach Anspruch 1, **dadurch gekennzeichnet, dass** das Magnetfeld (B) durch eine MRI-Anordnung (20) erzeugt wird.

3. Verfahren nach einem der Ansprüche 1 oder 2, **dadurch gekennzeichnet, dass** die Messung des Blutflusses während der Vornahme einer MRI-Diagnose erfolgt.

4. Verfahren nach einem der Ansprüche 1, 2 oder 3, **dadurch gekennzeichnet, dass** auf der Grundlage des ermittelten Signalverlaufs im Abschnitt (A) des Signals (E) der cardiac output bestimmt wird.

5. Vorrichtung (1, 10) zum nichtinvasiven Messen des EKG-Signales (E) eines Patienten (P), insbesondere zur Durchführung eines Verfahrens nach einem der Ansprüche 1 bis 4, mit einer EKG-Anordnung (1, 10) mit wenigstens zwei Elektroden (2) zum Messen einer Potentialdifferenz (ΔV) zwischen wenigstens zwei Stellen (P1, P2) der Oberfläche (H) des Körpers und zum Erzeugen eines Potentialsignals (E),
**dadurch gekennzeichnet, dass**
die Vorrichtung (1, 10) Mittel zum Ermitteln oder Mittel zum Ermitteln und Unterdrücken eines aufgrund des Halleffekts durch die Bewegung von Ladungsträgern (Q) im Gefäss (G) erzeugten Anteils (T) des Potentialsignals (E) aufweist.

6. Vorrichtung nach Anspruch 5, **dadurch gekennzeichnet, dass** die Vorrichtung Mittel zum automatischen Bestimmen des Blutflusses aufgrund des bestimmten Anteils (T) des Potentialsignals (E) aufweist.

## Claims

1. A method for recording and processing an ECG signal (E) of a patient (P), who is subjected to a magnetic field (B), comprising the steps.
- recording of a signal (E)
- determining a section (A) of the signal path, in which a signal component (T) is superimposed upon the signal (E), which component T is generated due to the Hall effect by charge carriers (Q) in the blood discharged through the heart, and
- processing the signal (E), **characterised by** the elimination of the signal component (T) of the signal (E) generated by the Hall effect in the determined section (A).

2. The method according to Claim 1, **characterised in that** the magnetic field (B) is generated by an MRI device (20).

3. The method according to one of Claims 1 or 2, **characterised in that** the blood flow is measured during the execution of an MRI diagnosis.

4. The method according to one of Claims 1, 2 or 3, **characterised in that** the cardiac output is determined on the basis of the determined signal path in section (A) of the signal (E).

5. A device (1, 10) for the non-invasive measurement of the ECG signal (E) of a patient (P), in particular for implementing a method according to one of Claims 1 to 4, with an ECG device (1, 10) with at least two electrodes (2) for measuring a potential difference (ΔV) between at least two points (P1, P2) on the surface (H) of the body and for generating a potential signal (E), **characterised in that** the device (1, 10) has means for determining or means for determining and suppressing a component (T) of the potential signal (E) generated due to the Hall effect by the movement of charge carriers (Q) in the vessel (G).

6. The device according to Claim 5, **characterised in that** the device has means for automatically determining the blood flow due to the determined component (T) of the potential signal (E).

## Revendications

1. Procédé pour enregistrer et traiter un signal d'électrocardiogramme (E) d'un patient (P) qui est exposé à un champ magnétique (B), composé des étapes suivantes:
- enregistrement d'un signal (E),
- détermination d'une section (A) de la courbe de signal dans laquelle s'ajoute au signal (E) une partie de signal (T) qui est produite en raison de l'effet Hall par des porteurs de charge (Q) dans le sang expulsé du coeur, et
- traitement du signal (E), **caractérisé par** la suppression, dans la section (A) déterminée, de la partie (T) du signal (E) provoquée par l'effet Hall.

2. Procédé selon la revendication 1, **caractérisé en ce que** le champ magnétique (B) est produit par un dispositif d'IRM (20) .

3. Procédé selon la revendication 1 ou 2, **caractérisé en ce que** la mesure du flux sanguin a lieu pendant qu'on procède à un diagnostic IRM.

4. Procédé selon l'une des revendications 1, 2 ou 3, **caractérisé en ce que** le débit cardiaque est défini sur la base de la courbe de signal déterminée, dans la section (A) du signal (E).

5. Dispositif (1, 10) pour une mesure non invasive du signal d'électrocardiogramme (E) d'un patient (P), en particulier pour la mise en oeuvre d'un procédé selon l'une des revendications 1 à 4, comprenant un électrocardiographe (1, 10) avec au moins deux électrodes (2) pour mesurer une différence de potentiel (ΔV) entre au moins deux endroits (P1, P2) de la surface (H) du corps et pour produire un signal de potentiel (E), **caractérisé en ce que** le dispositif (1, 10) comporte des moyens pour déterminer ou des moyens pour déterminer et supprimer une partie (T) du signal de potentiel (E) produite dans le vaisseau (G) par le déplacement des porteurs de charge (Q) à cause de l'effet Hall.

6. Dispositif selon la revendication 5, **caractérisé en ce qu'**il comporte des moyens pour définir automatiquement le flux sanguin à partir de la partie (T) définie du signal de potentiel (E).
